# FASCICULE DE BREVET EUROPEEN

(11) **EP 1 082 952 B1**
(45) Date de publication et mention de la délivrance du brevet: **10.07.2002**
(21) Numéro de dépôt: 00402186.1
(22) Date de dépôt: 28.07.2000
(51) Int. Cl.: A61K 7/00

(54) **Composition et procédé de maquillage des matières kératiniques**
Zusammensetzung und Schminkverfahren für keratinfasern
Composition and make-up method for keratin fibers

(30) Priorité: 07.09.1999 FR 9911174
(43) Date de publication de la demande: 14.03.2001
(73) Titulaire: L'OREAL, 75008 Paris (FR)
(72) Inventeur: Ramin, Roland, 75014 Paris (FR); Brenne, Ingrid, 94240 L'Hay Les Roses (FR)
(74) Mandataire: Kromer, Christophe

(56) Documents cités:
- EP-A- 0 327 739
- EP-A- 0 665 004
- US-A- 5 624 486
- PATENT ABSTRACTS OF JAPAN vol. 012, no. 372 (C-533), 5 octobre 1988 (1988-10-05) & JP 63 122616 A (YUJI UENISHI), 26 mai 1988 (1988-05-26)
- PATENT ABSTRACTS OF JAPAN vol. 015, no. 332 (C-0861), 23 août 1991 (1991-08-23) & JP 03 127708 A (KOA GLASS KK), 30 mai 1991 (1991-05-30)

## Description

La présente invention se rapporte à une composition cosmétique de maquillage des matières kératiniques comprenant des particules de verre traitées. L'invention a aussi pour objet un procédé cosmétique de maquillage des matières kératiniques. La composition selon l'invention peut être appliquée sur les ongles, la peau aussi bien du visage que du corps, y compris les lèvres, les cils et sur les cheveux, notamment d'êtres humains. Plus spécialement, l'invention porte sur un vernis à ongles.

La composition de maquillage peut être un vernis à ongles, un fard à joue ou à paupières, un fond de teint, un mascara, un eye-liner, un produit de maquillage pour les lèvres ou un produit de maquillage du corps. La composition peut également être appliquée sur les accessoires de maquillage comme les faux ongles, faux cils, postiches ou encore sur des pastilles ou des patchs adhérents sur la peau ou les lèvres (du type mouches).

Les compositions de maquillage, comme les vernis à ongles, sont colorées à l'aide de matières colorantes comme les colorants solubles, les pigments qui sont généralement des oxydes métalliques, tels que les oxydes de fer, ou bien encore à l'aide de nacres tels que les micas recouverts d'oxyde métalliques comme l'oxyde de titane. Toutefois, lorsque le film de maquillage est soumis à des contraintes tels que des chocs, des pressions ou des frottements, les matières colorantes et notamment les pigments ne sont pas toujours résistants à ces contraintes et s'usent. On constate alors une perte sensible de la couleur du maquillage, ce qui contraint l'utilisatrice à renouveler régulièrement l'application du maquillage, éventuellement après avoir retiré la couche abîmée. Il est également connu d'employer des paillettes d'aluminium pour obtenir une couleur à effet métallique. Ces paillettes sont comme les pigments peu résistantes aux contraintes et s'usent. De plus, ces paillettes sont opaques à la lumière, diffusantes et mates. En outre, lorsqu'elles sont associées avec des pigments colorés ne présentant pas d'aspect métallique, par exemples des nacres, la couleur de ces pigments ne ressort pas ; il n'est donc pas possible d'obtenir un maquillage ayant un aspect métallique coloré.
Il est connu du document US-A-5624486 des plaquettes silicates recouvertes d'une première couche d'oxyde métallique et d'une deuxième couche de métal et/ou d'oxyde métallique. Ces plaquettes sont utilisées pour des revêtements colorés, des encres, des plastiques, des verres, des céramiques et des compositions cosmétiques décoratives.

Le but de la présente invention est de disposer d'une composition de maquillage permettant de remédier aux inconvénients évoqués précédemment et notamment d'obtenir un maquillage sur les matières kératiniques d'aspect métallique éclatant et présentant une bonne résistance à l'usure.

Le demandeur a constaté qu'un nouveau type de maquillage des matières kératiniques pouvait être obtenu en utilisant une composition cosmétique comprenant des particules de verres particulières.

De façon plus précise, l'invention a pour objet une composition cosmétique de maquillage comprenant dans un milieu cosmétiquement acceptable une matière colorante, caractérisée par le fait que la matière colorante comprend des particules de verre recouvertes d'au moins une couche métallique.

Lorsque la composition selon l'invention est appliquée sur les matières kératiniques tels que les ongles, les particules de verre recouvertes de la couche métallique se répartissent facilement dans la couche déposée. Cette composition procure un film de maquillage d'aspect métallique très brillant, éclatant, à effet miroir, quelle que soit la direction d'observation et ne diffusant pas la lumière. Le maquillage présente en outre une bonne résistance à l'usure, notamment aux chocs, aux frottements, à l'abrasion, ainsi qu'une bonne résistance à l'écaillage. On obtient ainsi un maquillage éclatant, de bonne adhérence sur le support maquillé et de bonne tenue. De plus, ces particules de verre transmettent bien la lumière, ce qui permet de les associer avec des pigments colorés pour obtenir un maquillage à effet métallique coloré.

Un autre objet de l'invention est un procédé cosmétique de maquillage des matières kératiniques comprenant l'application sur les matières kératiniques d'une composition telle que définie précédemment.

L'invention a également pour objet l'utilisation, dans une composition de maquillage des matières kératiniques, de particules de verre recouvertes d'au moins couche métallique pour obtenir un maquillage brillant et/ou résistant à l'usure.

Les particules de verre utilisées comme matière colorante dans la composition selon l'invention sont recouvertes d'au moins une couche métallique.

La couche métallique peut être formée d'au moins un métal choisi parmi l'argent, l'aluminium, le chrome, le nickel, le molybdène, l'or, le cuivre, l'étain, le magnésium et leurs mélanges (alliages). On utilise de préférence l'argent, le chrome, le nickel, le molybdène, et leurs mélanges.

La couche métallique des particules de verre recouvertes peut être présente en une teneur allant de 0,1 à 50 % en poids, par rapport au poids total des particules, de préférence de 1 % à 20 % en poids, et mieux de 2 % à 8 % en poids.

Les particules de verres recouvertes de la couche métallique peuvent avoir une taille moyenne allant de 1 µm à 500 µm, de préférence de 10 µm à 300 µm, et mieux de 25 µm à 150 µm.

Avantageusement, les particules de verres recouvertes de la couche métallique peuvent avoir une épaisseur allant de 100 nm à 25 µm, de préférence de 500 nm à 10 µm, et mieux de 0,5 µm à 5 µm.

Des particules de verre recouvertes de la couche métallique sont décrites notamment dans les documents JP-A-09188830, JP-A-10158450, JP-A-10158541, JP-A-07258460 et JP-A-05017710.

Comme particules de verre recouvertes d'une couche métallique, on peut utiliser par exemples les particules recouvertes d'argent vendues sous les dénominations Microglass Metashine REFSX 2025 PS, GF 2140 par la société TOYAL, les particules recouvertes d'alliage nickel/chrome/molybdène vendues sous les dénominations Crystal star GF 550, GF 2525 par la société TOYAL.

Les particules de verre recouvertes de la couche métallique peuvent être présentes dans la composition selon l'invention en une teneur allant de 0,1 % à 90 % en poids, par rapport au poids total de la composition, de préférence de 1 % à 30 % en poids, et mieux de 2 % à 10 % en poids.

Selon l'invention, la composition selon l'invention comprend un milieu cosmétiquement acceptable qui peut comprendre un milieu aqueux ou un milieu solvant organique.

La composition selon l'invention peut comprendre, en outre, au moins un polymère filmogène. Dans la présente demande, on entend par "polymère filmogène", un polymère apte à former à lui seul ou en présence d'un agent auxiliaire de filmification, un film isolable. Le polymère filmogène de la composition peut être solubilisé ou dispersé sous forme de particules dans le milieu cosmétiquement acceptable de la composition selon l'invention.

Parmi les polymères filmogènes utilisables dans les compositions de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation d'un ou plusieurs monomères à insaturation notamment éthylénique, certain monomère étant susceptible de s'homopolymériser (à l'inverse des polycondensats).
Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique, et leurs mélanges. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂ . Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique en dispersion aqueuse utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432® par la société DOW CHEMICAL.

On peut ainsi citer, parmi les polycondensats utilisables comme polymère filmogène, les polyuréthannes anioniques, cationiques, non-ioniques ou amphotères, les polyuréthannes-acryliques, les poly-uréthannes-polyvinylpirrolidones, les polyester-polyuréthannes, les polyéther-polyuréthannes, les polyurées, les polyurée/polyuréthannes, et leurs mélanges.
Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Comme polymère filmogène polyuréthane en dispersion aqueuse utilisable selon l'invention, on peut notamment citer les polyester-polyuréthanes vendus sous les dénominations "AVALURE UR-405®", "AVALURE UR-410®", "AVALURE UR-425®", "AVALURE UR-450®", "SANCURE 2060®" par la société GOODRICH et les polyéther-polyuréthanes vendus sous les dénominations "SANCURE 878®" par la société GOODRICH, "NEOREZ R 970®" par la société ZENECA.

Parmi les polycondensats filmogènes, on peut également citer les polyesters, les polyesters amides, les polyesters à chaîne grasse, les polyamides, et les résines époxyesters, les résines résultant de la condensation de formaldéhyde avec une arylsulfonamide, les résines aryl-sulfonamide époxy.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques tels que la nitrocellulose, l'acétate de cellulose, l'acétobutyrate de cellulose, l'acétopropionate de cellulose, l'éthyl cellulose, et leurs mélanges.

Le polymère filmogène peut être généralement présent dans la composition selon l'invention en une teneur allant de 1 % à 70 % en poids, par rapport au poids total de la composition, et mieux allant de 10 % à 40 % en poids.

Comme solvant organique utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol;
- les glycols liquides à température ambiante tels que l'éthylène glycol, le propylène glycol, le pentylène glycol, le glycérol;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

Ces solvants conviennent plus particulièrement pour le maquillage des ongles : la composition constitue alors un vernis à ongles.

La teneur en solvant dans la composition selon l'invention peut aller de 30 à 99 % en poids, par rapport au poids total de la composition, et de préférence de 60 % à 90 % en poids.
Le solvant peut être un solvant organique ou bien encore un milieu aqueux, ce dernier pouvant être constitué essentiellement d'eau ou bien encore d'un mélange hydroalcoolique et en particulier contenant des monoalcools inférieurs en C₁-C₅.

Pour améliorer les propriétés filmogènes de la composition, notamment de la composition de base et/ou de surface selon l'invention, un agent auxiliaire de filmification peut être prévu.

Lorsque l'on utilise un agent auxiliaire de filmification avec le polymère filmogène, l'agent auxiliaire de filmification peut être choisi parmi tous les composés connus de l'homme du métier comme étant susceptibles de remplir la fonction recherchée, et être notamment choisi parmi les agents plastifiants.

En outre, lorsque la composition comprend un polymère filmogène sous forme de particules dispersées dans le milieu correspondant de la composition, l'agent auxiliaire de filmification peut aussi être choisi parmi les agents de coalescence.

La composition selon l'invention peut en outre comprendre des matières colorantes additionnelles, différentes des particules de verre décrites précédemment. La matière colorante peut notamment être choisie parmi les colorants (hydrosolubles ou liposolubles) ou les matières colorantes pulvérulentes tels que les pigments, les nacres, les paillettes bien connues de l'homme du métier. Les matières colorantes additionnelles peuvent être présentes, dans la composition, en une teneur allant de 0,01 % à 50 % en poids, par rapport au poids de la composition, de préférence de 0,01 % à 30 % en poids.

La composition selon l'invention peut en outre comprendre tout additif connu de l'homme du métier comme étant susceptible d'être incorporé dans une telle composition, tels que les agents épaississants, les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peuvent être préparées par l'homme du métier sur la base de ses connaissances générales et selon l'état de la technique.

L'invention est illustrée plus en détail dans les exemples suivants.

### Exemple 1 :

On a préparé un vernis à ongles ayant la composition suivante:
- Nitrocellulose 10 g
- Plastifiants et résine 15 g
- Agent rhéologique 1,5 g
- Particules de verre recouvertes d'argent (GF 2140 de la société TOYAL) 10 g
- Acétate d'éthyle, acétate de butyle qsp 100 g

Après application de la composition sur les ongles, on a obtenu un film de maquillage présentant un aspect métallique très brillant. Le maquillage est bien résistant à l'usure.

### Exempte 2 :

On a préparé un vernis à ongles dont la composition est la suivante :
- Nitrocellulose 10 g
- Plastifiants et résine 15 g
- Agent rhéologique 1,5 g
- Particules de verre recouvertes d'alliage nickel/chrome/molybdène (CRYSTAL STAR GF 550 de la société TOYAL) 5 g
- Pigment rouge 0,5 g
- Acétate d'éthyle, acétate de butyle qsp 100 g

La composition est appliquée sur les ongles. On obtient un maquillage de couleur rouge présentant un aspect métallique éclatant.

### Exemples 3 à 5 comparatifs :

On a préparé 2 vemis à ongles selon l'invention (exemples 3 et 4) et un vernis à ongles ne faisant pas partie de l'invention (exemple 5) selon les compositions suivantes, les teneurs étant indiquées en gramme :

| **Ingrédient** | **Exemple 3** | **Exemple 4** | **Exemple 5** |
|---|---|---|---|
| Nitrocellulose | 11 | 11 | 11 |
| Copolymère tosylamide/formaldéhyde | 10,1 | 10,1 | 10,1 |
| Bentonite | 1,1 | 1,1 | 1,1 |
| Acetyl citrate de tributyle | 6,7 | 6,7 | 6,7 |
| Alcool isopropylique | 7,9 | 7,9 | 7,9 |
| Pigment (1) | 10 | - | - |
| Pigment (2) | - | 10 | - |
| Poudre d'aluminium (3) | - | - | 10 |
| Acétate d'éthyle | 22 | 22 | 22 |
| Acétate de butyle | 39,2 | 39,2 | 39,2 |

| | | | |
|---|---|---|---|
| (1) : Particules de verre recouvertes d'argent vendues sous la dénomination GF 2140 par la société TOYAL | | | |
| (2) : Particules de verre recouvertes d'argent vendues sous la dénomination MICROGLASS METASHINE REFSX 2025PS par la société TOYAL | | | |
| (3) : Poudre d'aluminium vendue sous la dénomination SILVET ET 1630 par la société SILBERLINE | | | |

### a) Test de résistance à l'usure :

La résistance à l'usure des films est mesurée selon la norme AFNOR NF T 30-015.
On a appliqué chaque composition sous forme d'une couche de 600 µm d'épaisseur (avant séchage) sur un disque puis laisser sécher pendant 1 heure à 30 °C. Le film de vernis déposé sur le disque a ensuite été mis pendant 1 heure en contact avec des disques abrasifs (abrasimètre TABER), le disque faisant une rotation complète en 1 seconde. Au bout d'une heure, on a pesé le disque et calculé la perte de masse PM de produit exprimée en pourcentage du poids perdu par rapport au poids initial.

On a obtenu les résultats suivants :
Exemple 3 : PM =3,58 %
Exemple 4 : PM = 4,98 %
Exemple 5 : PM = 5, 99 %

On constate que les compositions 3 et 4 selon l'invention ont une meilleure résistance à l'abrasion que la composition 5.

### b) Test de réflexion lumineuse :

Les compositions testées contiennent 5 % en poids de pigment respectivement (1), (2) ou (3) au lieu de 10 %.

Pour chaque composition, on a déposé sur plaque de verre une couche de 300 µm d'épaisseur (avant séchage), puis après séchage pendant 24 heures à température ambiante, on a déterminé les propriétés de réflexion de la lumière du film de vernis.

On a éclairé le film avec un faisceau lumineux de 1 cm de diamètre incliné à un angle de 30 °par rapport à la normale au plan de la plaque de verre puis on a mesuré à l'aide d'un photogoniomètre à angle variable, la quantité de lumière réfléchie en fonction de l'angle.

Pour chaque composition, la courbe de réflexion présente les caractéristiques suivantes :

| | **Exemple 3** | **Exemple 4** | **Exemple 5** |
|---|---|---|---|
| Maximum du pic (en unité arbitraire, à l'angle spéculaire -30°) | 253,7 | 451,6 | 173,1 |
| Largeur à mi-hauteur (en ° d'angle) | 12,5 | 14 | 21 |

On constate que les courbes de réflexion des films obtenus avec les compositions des exemples 3 et 4 selon l'invention ont un maximum plus élevé et une largeur à mi-hauteur plus étroite que la courbe de réflexion du film de l'exemple 5. Les films des exemples 3 et 4 réfléchissent donc beaucoup plus la lumière que le film de l'exemple 5. En outre, les films des exemples 3 et 4 diffusent moins la lumière que le film de l'exemple 5 car la divergence du faisceau réfléchi est plus faible pour les films des exemples 3 et 4 que celle du film de l'exemple 5. Les particules de verre recouvertes des exemples 3 et 4 permettent donc d'obtenir un maquillage plus brillant et plus scintillant que le maquillage de l'exemple 5 obtenu avec la poudre d'aluminium qui est beaucoup plus mat.

### c) Test de transmission lumineuse :

Sur les mêmes films utilisés pour le test de réflexion lumineuse décrit précédemment, on a éclairé chaque film avec un faisceau lumineux orientés perpendiculairement au film (angle de 90 ° par rapport au plan de la plaque de verre). On a mesuré la quantité de lumière ayant traversé le film en fonction de l'angle. On a mesuré pour l'angle de - 90 ° la transmission "t" de la lumière et obtenu les valeurs arbitraires suivantes :
Exemple 3 : t = 188,6
Exemple 4 : t = 114,9
Exemple 5 : t = 0

On constate que les films des exemples 3 et 4 sont de bons transmetteurs de la lumière et non diffusant tandis que le film de l'exemple 5, ne transmettant pas la lumière, est opaque.

## Revendications

1. Composition cosmétique de maquillage comprenant dans un milieu cosmétiquement acceptable une matière colorante, **caractérisée par le fait que** la matière colorante comprend au moins des particules de verre recouvertes d'au moins une couche métallique.

2. Composition selon la revendication 1, **caractérisée par le fait que** la couche métallique est formée d'au moins un métal choisi dans le groupe formé par l'argent, le nickel, le chrome, le molybdène, l'aluminium, l'or, le cuivre, l'étain, le magnésium, et leurs mélanges.

3. Composition selon la revendication 1 ou 2, **caractérisée par le fait que** la couche métallique est formée d'au moins un métal choisi dans le groupe formé par l'argent, le nickel, le chrome, le molybdène, et leurs mélanges.

4. Composition selon l'une quelconque des revendications précédentes, **caractérisée par le fait que** la couche métallique est présente dans les particules de verre en une teneur allant de 0,1 à 50 % en poids, par rapport au poids total des particules, et de préférence de 2 % à 8 % en poids.

5. Composition selon l'une quelconque des revendications précédentes, **caractérisée par le fait que** les particules de verres recouvertes de la couche métallique ont une taille moyenne allant de 1 µm à 500 µm, de préférence de 10 µm à 300 µm, et mieux de 25 µm à 150 µm.

6. Composition selon l'une quelconque des revendications précédentes, **caractérisée par le fait que** les particules de verre recouvertes de la couche métallique ont une épaisseur allant de 100 nm à 25 µm, de préférence de 500 nm à 10 µm, et mieux de 0,5 µm à 5 µm.

7. Composition selon l'une quelconque des revendications précédentes, **caractérisée par le fait que** les particules de verre recouvertes de la couche métallique sont présents en une teneur allant de 0,1 % à 90 % en poids, par rapport au poids total de la composition, de préférence de 1 % à 30 % en poids, et mieux de 2 % à 10 % en poids.

8. Composition selon l'une quelconque des revendications précédentes, **caractérisée par le fait qu'**elle comprend un milieu solvant organique ou un milieu aqueux.

9. Composition selon l'une quelconque des revendications précédentes, **caractérisée par le fait qu'**elle comprend, en outre, au moins un polymère filmogène.

10. Composition selon la revendication 9, **caractérisée par le fait que** le polymère filmogène est choisi dans le groupe formé par les polymères radicalaires, les polycondensats et les polymères d'origine naturelle.

11. Composition selon l'une des revendications 9 ou 10, **caractérisée par le fait que** le polymère filmogène est choisi dans le groupe formé par les polymères vinyliques, les polyuréthanes, les polyesters, les polymères cellulosiques.

12. Composition selon l'une quelconque des revendications 9 à 11, **caractérisée par le fait que** la teneur en polymère filmogène va de 1 % à 70 % en poids, par rapport au poids total de la composition, et de préférence de 10 % à 40 % en poids.

13. Composition selon l'une quelconque des revendications précédentes, **caractérisée par le fait que** la matière colorante comprend en outre une matière colorante additionnelle différente de celle des particules de verre recouvertes de la couche métallique.

14. Composition selon la revendication 13, **caractérisée par le fait que** la matière colorante additionnelle est choisie parmi les colorants et les matières colorantes pulvérulentes.

15. Composition selon l'une quelconque des revendications précédentes, **caractérisée par le fait qu'**elle comprend en outre au moins un additif choisi dans le groupe formé par les agents auxiliaires de filmification, les agents épaississants, les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

16. Composition selon l'une quelconque des revendications précédentes, **caractérisée par le fait qu'**elle se présente sous la forme de vernis à ongles, de fard à joue ou à paupières, de fond de teint, de mascara, de produit de maquillage pour les lèvres, d'eye-liner ou de produit de maquillage du corps.

17. Procédé cosmétique de maquillage des matières kératiniques, consistant à appliquer sur les matières kératiniques au moins une couche d'une composition selon l'une quelconque des revendications précédentes.

18. Support maquillé comprenant un maquillage susceptible d'être obtenu selon le procédé tel que défini selon la revendication 17.

19. Support selon la revendication 18, **caractérisé par le fait qu'**il se présente sous forme de faux ongles, de faux cils ou de postiches.

20. Utilisation, dans une composition de maquillage des matières kératiniques, de particules de verre recouvertes d'au moins une couche métallique pour obtenir un maquillage brillant et/ou résistant à l'usure.

## Patentansprüche

1. Kosmetische Zusammensetzung zum Schminken, die in einem kosmetisch akzeptablen Medium ein Farbmittel enthält, **dadurch gekennzeichnet, dass** das Farbmittel zumindest Glaspartikel umfasst, die mit mindestens einer metallischen Schicht überzogen sind.

2. Zusammensetzung nach Anspruch 1, **dadurch gekennzeichnet, dass** die metallische Schicht von mindestens einem Metall gebildet wird, das unter Silber, Nickel, Chrom, Molybdän, Aluminium, Gold, Kupfer, Zinn, Magnesium und deren Gemischen ausgewählt ist.

3. Zusammensetzung nach Anspruch 1 oder 2, **dadurch gekennzeichnet, dass** die metallische Schicht von mindestens einem Metall gebildet wird, das unter Silber, Nickel, Chrom, Molybdän und deren Gemischen ausgewählt ist.

4. Zusammensetzung nach einem der vorhergehenden Ansprüche, **dadurch gekennzeichnet, dass** die metallische Schicht in den Glaspartikeln in einem Mengenanteil von 0,1 bis 50 Gew.-% und vorzugsweise von 2 bis 8 Gew.-%, bezogen auf das Gesamtgewicht der Partikel, vorliegt.

5. Zusammensetzung nach einem der vorhergehenden Ansprüche, **dadurch gekennzeichnet, dass** die mit der metallischen Schicht überzogenen Glaspartikel eine mittlere Größe von 1 µm bis 500 µm, vorzugsweise von 10 µm bis 300 µm und besser noch von 25 µm bis 150 µm aufweisen.

6. Zusammensetzung nach einem der vorhergehenden Ansprüche, **dadurch gekennzeichnet, dass** die mit der metallischen Schicht überzogenen Glaspartikel eine Dicke von 100 nm bis 25 µm, vorzugsweise von 500 nm bis 10 µm und besser noch von 0,5 µm bis 5 µm aufweisen.

7. Zusammensetzung nach einem der vorhergehenden Ansprüche, **dadurch gekennzeichnet, dass** die mit der metallischen Schicht überzogenen Glaspartikel in einem Mengenanteil im Bereich von 0,1 bis 90 Gew.-%, vorzugsweise von 1 bis 30 Gew.-% und besser noch von 2 bis 10 Gew.-%, bezogen auf das Gesamtgewicht der Zusammensetzung, vorliegen.

8. Zusammensetzung nach einem der vorhergehenden Ansprüche, **dadurch gekennzeichnet, dass** sie ein organisches Lösemittelmedium oder ein wässeriges Medium enthält.

9. Zusammensetzung nach einem der vorhergehenden Ansprüche, **dadurch gekennzeichnet, dass** sie ferner mindestens ein filmbildendes Polymer enthält.

10. Zusammensetzung nach Anspruch 9, **dadurch gekennzeichnet, dass** das filmbildende Polymer unter durch radikalische Polymerisation hergestellten Polymeren, Polykondensaten und Polymeren natürlichen Ursprungs ausgewählt ist.

11. Zusammensetzung nach einem der Ansprüche 9 oder 10, **dadurch gekennzeichnet, dass** das filmbildende Polymer unter Vinylpolymeren, Polyurethanen, Polyestern und Cellulosepolymeren ausgewählt ist.

12. Zusammensetzung nach einem der Ansprüche 9 bis 11, **dadurch gekennzeichnet, dass** der Mengenanteil des filmbildenden Polymers im Bereich von 1 bis 70 Gew.-% und vorzugsweise von 10 bis 40 Gew.-%, bezogen auf das Gesamtgewicht der Zusammensetzung, liegt.

13. Zusammensetzung nach einem der vorhergehenden Ansprüche, **dadurch gekennzeichnet, dass** das Farbmittel ferner ein zusätzliches Farbmittel umfasst, das von dem Farbmittel der mit der metallischen Schicht überzogenen Glaspartikel verschieden ist.

14. Zusammensetzung nach Anspruch 13, **dadurch gekennzeichnet, dass** das zusätzliche Farbmittel unter Farbstoffen und pulverförmigen Farbmitteln ausgewählt ist.

15. Zusammensetzung nach einem der vorhergehenden Ansprüche, **dadurch gekennzeichnet, dass** sie ferner mindestens einen Zusatzstoff enthält, der unter Hilfsmitteln für die Filmbildung, Verdickungsmitteln, Füllstoffen, Mitteln zur Verteilung, Netzmitteln, Dispergiermitteln, Schauminhibitoren, Konservierungsstoffen, UV-Filtern, Wirkstoffen, grenzflächenaktiven Stoffen, Hydratisierungsmitteln, Parfums, Neutralisationsmitteln, Stabilisatoren und Antioxidantien ausgewählt ist.

16. Zusammensetzung nach einem der vorhergehenden Ansprüche, **dadurch gekennzeichnet, dass** sie in Form eines Nagellacks, eines Wangenrouges, eines Lidschattens, eines Make-ups, einer Mascara, eines Schminkprodukts für die Lippen, eines Eyeliners oder eines Produkts zum Schminken des Körpers vorliegt.

17. Kosmetisches Verfahren zum Schminken von Keratinsubstanzen, das darin besteht, dass mindestens eine Schicht einer Zusammensetzung nach einem der vorhergehenden Ansprüche auf die Keratinsubstanzen aufgebracht wird.

18. Geschminkter Träger, der eine Schminke umfasst, die nach dem gemäß Anspruch 17 definierten Verfahren erhalten werden kann.

19. Träger nach Anspruch 18, **dadurch gekennzeichnet, dass** er in Form von falschen Nägeln, falschen Wimpern oder Perücken vorliegt.

20. Verwendung von Glaspartikeln, die mit mindestens einer metallischen Schicht überzogen sind, in einer Zusammensetzung zum Schminken von Keratinsubstanzen, um eine glänzende und/oder gegenüber Abnutzung beständige Schminke zu erhalten.

## Claims

1. Cosmetic make-up composition comprising a dyestuff in a cosmetically acceptable medium, **characterized in that** the dyestuff comprises at least glass particles coated with at least one metallic coat.

2. Composition according to Claim 1, **characterized in that** the metallic coat is formed from at least one metal chosen from the group formed by silver, nickel, chromium, molybdenum, aluminium, gold, copper, tin and magnesium, and mixtures thereof.

3. Composition according to Claim 1 or 2, **characterized in that** the metallic coat is formed from at least one metal chosen from the group formed by silver, nickel, chromium and molybdenum, and mixtures thereof.

4. Composition according to any one of the preceding claims, **characterized in that** the metallic coat is present in the glass particles in a content ranging from 0.1% to 50% by weight, relative to the total weight of the particles, and preferably from 2% to 8% by weight.

5. Composition according to any one of the preceding claims, **characterized in that** the glass particles coated with the metallic coat have an average size ranging from 1 µm to 500 µm, preferably from 10 µm to 300 µm and better still from 25 µm to 150 µm.

6. Composition according to any one of the preceding claims, **characterized in that** the glass particles coated with the metallic coat have a thickness ranging from 100 nm to 25 µm, preferably from 500 nm to 10 µm and better still from 0.5 µm to 5 µm.

7. Composition according to any one of the preceding claims, **characterized in that** the glass particles coated with the metallic coat are present in a content ranging from 0.1% to 90% by weight, relative to the total weight of the composition, preferably from 1% to 30% by weight, and better still, from 2% to 10% by weight.

8. Composition according to any one of the preceding claims, **characterized in that** it comprises an organic solvent medium or an aqueous medium.

9. Composition according to any one of the preceding claims, **characterized in that** it also comprises at least one film-forming polymer.

10. Composition according to Claim 9, **characterized in that** the film-forming polymer is chosen from the group formed by radical-mediated polymers, polycondensates and polymers of natural origin.

11. Composition according to either of Claims 9 and 10, **characterized in that** the film-forming polymer is chosen from the group formed by vinyl polymers, polyurethanes, polyesters and cellulose polymers.

12. Composition according to any one of Claims 9 to 11, **characterized in that** the content of film-forming polymer ranges from 1% to 70% by weight, relative to the total weight of the composition, and preferably from 10% to 40% by weight.

13. Composition according to any one of the preceding claims, **characterized in that** the dyestuff also comprises an additional dyestuff other than that of the glass particles coated with the metallic coat.

14. Composition according to Claim 13, **characterized in that** the additional dyestuff is chosen from dyes and pulverulent dyestuffs.

15. Composition according to any one of the preceding claims, **characterized in that** it also comprises at least one additive chosen from the group formed by auxiliary film-forming agents, thickeners, fillers, spreading agents, wetting agents, dispersants, anti-foaming agents, preserving agents, UV screening agents, active agents, surfactants, moisturizers, fragrances, neutralizing agents, stabilizers and antioxidants.

16. Composition according to any one of the preceding claims, **characterized in that** it is in the form of a nail varnish, a face powder, an eyeshadow, a foundation, a mascara, a make-up product for the lips, an eyeliner or a make-up product for the body.

17. Cosmetic process for making up keratin substances, which consists in applying at least one coat of a composition according to any one of the preceding claims to the keratin substances.

18. Support to which make-up has been applied, comprising a make-up which can be obtained according to the process as defined according to Claim 17.

19. Support according to Claim 18, **characterized in that** it is in the form of false nails, false eyelashes or wigs.

20. Use, in a composition for making up keratin substances, of glass particles coated with at least one metallic coat, in order to obtain a glossy and/or wear-resistant make-up.
